(19) Europäisches Patentamt / European Patent Office / Office européen des brevets

(11) **EP 4 763 863 A1**

(12) **EUROPEAN PATENT APPLICATION**
published in accordance with Art. 153(4) EPC

(43) Date of publication:
**24.06.2026 Bulletin 2026/26**

(21) Application number: **24876136.3**

(22) Date of filing: **04.07.2024**

(51) International Patent Classification (IPC):
*C07K 16/28* (2006.01)    *C07K 16/30* (2006.01)
*C12N 15/13* (2006.01)    *A61K 39/395* (2006.01)
*A61P 35/00* (2006.01)    *A61P 35/02* (2006.01)
*G01N 33/574* (2006.01)

(86) International application number:
**PCT/CN2024/103547**

(87) International publication number:
**WO 2025/077315 (17.04.2025 Gazette 2025/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.10.2023   CN 202311320141**

(71) Applicant: **Carbiogene Therapeutics Co., Ltd.
Hangzhou, Zhejiang 310051 (CN)**

(72) Inventors:
• **ZHU, Jiangao
Hangzhou, Zhejiang 310051 (CN)**

• **YANG, Xin
Hangzhou, Zhejiang 310051 (CN)**
• **YANG, Wenjun
Hangzhou, Zhejiang 310051 (CN)**

(74) Representative: **Lorenz Seidler Gossel Part. mbB
Widenmayerstr. 23
80538 München (DE)**

Remarks:
The complete document including Reference Table(s) and the Sequence Listing(s) can be downloaded from the EPO website

(54) **ANTI-BCMA SINGLE-DOMAIN ANTIBODY, AND PREPARATION METHOD THEREFOR AND USE THEREOF**

(57)    Provided are an anti-BCMA single-domain antibody, and a preparation method therefor and the use thereof. Specifically, provided is a single-domain antibody having an amino acid sequence of SEQ ID No. 1. The single-domain antibody has high affinity, can thoroughly specifically target BCMA-positive cells, and can be applied to the detection of BCMA expression in bone marrow cells of MM patients. The single-domain antibody can be prepared into a specific antibody drug clinically used for preventing and treating BCMA-target-related diseases (such as multiple myeloma, B-cell acute lymphoblastic leukemia, non-Hodgkin's lymphoma and Hodgkin's lymphoma); or a BCMA protein detection kit, etc. The single-domain antibody has a stable structure, a small molecular size, is easily recombinantly expressed and has a low production cost, can be used alone or as a drug delivery system to carry relevant drugs, and has very wide prospects and important significance in fields such as drug application and clinical diagnosis.

EP 4 763 863 A1

**Description**

**CROSS-REFERENCE TO RELATED APPLICATION**

The present application claims priority to Chinese Patent Application No. 202311320141.7, entitled "ANTI-BCMA SINGLE-DOMAIN ANTIBODY, AND PREPARATION METHOD THEREFOR AND USE THEREOF", filed on October 12, 2023, to China National Intellectual Property Administration, the entire contents of which are hereby incorporated by reference herein.

**Technical Field**

The present application belongs to the field of biomedicine, and specifically relates to an anti-BCMA single-domain antibody, and a preparation method therefor and use thereof.

**Background Art**

Multiple myeloma (MM) is a malignant disease of abnormal proliferation of monoclonal plasma cells, which is a common malignant tumor in the hematological system. The common symptoms of MM include myeloma-related organ dysfunction and secondary amyloidosis etc. The Chinese Guideline for Diagnosis and Treatment of Multiple Myeloma (revised in 2022) recommends that autologous hematopoietic stem cell transplantation (ASCT) is the first choice for multiple myeloma patients with a good general physical status score. For young patients with high-risk prognostic factors and suitable donors, allogeneic hematopoietic stem cell transplantation (Allo-SCT) can be considered. In addition, a chimeric antigen receptor T cell (CAR-T) (clinical trial, investigator-initiated study) treatment regimen may also be used in relapsed patients. Currently, CAR-T therapy targeting B-cell maturation antigen (BCMA) is clinically effective in multiple myeloma. BCMA (CD269, TNFRSF17) is a type III transmembrane glycoprotein belonging to the human tumor necrosis factor receptor (TNFR) superfamily, which plays a major role in the maturation and differentiation of B cells into plasma cells. BAFF and APRIL are two B-cell activation and expansion-inducing ligands that bind to BCMA and together regulate B-cell function. BCMA is mainly expressed in mature B cells, and its expression gradually increases with the maturation of B cells, and is selectively overexpressed in plasma cells during malignant transformation, which promotes tumor cell growth, survival, and the development of drug resistance. BCMA is highly expressed on a variety of tumor cells, but not expressed or expressed at low levels in other normal tissues, which is an ideal target for cancer-targeted therapy.

Heavy chain antibody (HcAb) is a naturally occurring antibody type, which is a unique antibody type found in camelids or cartilaginous fish. Its antibody domain naturally lacks a light chain and is composed of only two heavy chains. The antigen recognition function of heavy chain antibodies is primarily determined by the variable region (VHH) of the heavy chain antibody. VHH alone can recognize antigens and is therefore also referred to as a single-domain antibody (sdAb). A single-domain antibody lacks an antibody light chain and has only a heavy chain variable region, which is also referred to as a Nanobody (Nb) due to its small molecular weight. The single-domain antibody has a molecular weight of only about 13-15 KDa, a diameter of about 2.5 nm, and a length of 4 nm, and is the smallest antibody fragment with antigen-binding function so far. Its antigen-binding ability and stability are basically the same as those of the intact antibody, or have a higher specific antigen affinity. Compared with traditional antibodies, single-domain antibodies also have many unique properties, such as better tissue permeability, higher thermal and chemical stability, ability to reach specific epitopes, arbitrary combination in a building block mode, and low production cost etc. Conventional methods for obtaining single-domain antibodies consist of numerous steps, including multiple immunizations of camelids, isolation of B lymphocytes, amplification of the VHH region, construction and screening of display libraries, etc. With the development of synthetic biology, it is possible to construct a high-quality randomized large-capacity single-domain antibody library based on total synthesis. At present, VHH single-domain antibody has been widely used in the research of miniaturized genetic engineering antibodies, the development of new drugs, and the diagnosis and treatment of diseases due to its advantages, such as stable structure, small molecular size, good solubility, good tissue permeability, easy recombinant expression, high homology with human antibodies, easy humanization, and low immunogenicity. Single-domain antibodies have a wide range of applications in biomedical research and medical treatment.

**Summary of the Invention**

It is an object of the present application to provide a single-domain antibody targeting the BCMA antigen and the use thereof in tumor-targeted therapy. In the present application, an alpaca was immunized with a BCMA recombinant protein to obtain a single-domain antibody gene, and a single-domain antibody expression library was constructed. And then a high-affinity anti-BCMA single-domain antibody (BCMA-VHH-54) was screened from the single-domain antibody expression library by phage display screening technology. After sequencing the antibody gene sequence, BCMA-VHH-54 was prepared by a genetic engineering method. Furthermore, functionality experiments such as affinity and specificity were performed on the obtained antibody. The results showed that the single-domain antibody of the present application had high affinity, could specifically target BCMA-positive cells (cells expressing BCMA protein), and could be successfully used in detecting BCMA expression in bone marrow cells of MM patients. The single-domain antibody of the present application can be expressed and produced in prokaryotic cells, yeast cells, eukaryotic cells, and any recombinant system, and can be prepared into a specific antibody drug clinically used for preventing and treating BCMA-target-related diseases (multiple myeloma, acute B-lymphoblastic leukemia, non-Hodgkin lymphoma, and Hodgkin lymphoma); or a BCMA protein detection kit, etc. The single-domain antibody has a stable structure, a small molecular size, is easily recombinantly expressed, and has a low production cost. It can be used alone or as a drug delivery system to carry relevant drugs, and has very wide prospects and important significance in fields such as drug application and clinical diagnosis.

**Technical Problem**

It is an object of the present application to provide a single-domain antibody targeting the BCMA antigen and the use thereof in tumor-targeted therapy.

**Technical Solution**

To achieve the above object, the present application firstly provides a single-domain antibody, named BCMA-VHH-54, which can be composed of a heavy chain variable region (VHH), wherein the heavy chain variable region can include the amino acid sequences of the complementarity determining region CDR1 at positions 26-33 of SEQ ID No.1, the complementarity determining region CDR2 at positions 51-58 of SEQ ID No.1 and the complementarity determining region CDR3 at positions 97-106 of SEQ ID No.1, respectively.

The single-domain antibody (anti-BCMA single-domain antibody) can be a single-domain antibody that specifically binds to the BCMA protein.

Further, the amino acid sequence of the heavy chain variable region can be SEQ ID No.1 or an amino acid sequence that is at least 80% identical to SEQ ID No.1 and has the same function.

The single-domain antibody BCMA-VHH-54 has only the variable region (VHH) of the heavy chain antibody, and in turn consists of framework region FR1, complementarity determining region CDR1, framework region FR2, complementarity determining region CDR2, framework region FR3, complementarity determining region CDR3, and framework region FR4.

The amino acid sequence of the single-domain antibody BCMA-VHH-54 can be SEQ ID No.1. In SEQ ID No.1, positions 1-25 are framework region FR1, positions 26-33 are complementarity determining region CDR1, positions 34-50 are framework region FR2, positions 51-58 are complementarity determining region CDR2, positions 59-96 are framework region FR3, positions 97-106 are complementarity determining region CDR3, and positions 107-117 are framework region FR4. The nucleotide sequence of the nucleic acid molecule (BCMA-VHH-54 gene) encoding the single-domain antibody BCMA-VHH-54 is shown in SEQ ID No.2.

The sequence of the complementarity determining regions is defined according to the Kabat numbering system.

Other variants of the single-domain antibody sequences of the present application having improved affinity and/or potency may be obtained by using methods known in the art and are included within the scope of the present application. For example, amino acid substitutions can be used to obtain antibodies with further improved affinity. Alternatively, codon optimization of the nucleotide sequence may be used to improve translation efficiency in the expression system used to produce the antibody. In addition, polynucleotides including sequences that optimize antibody specificity by applying directed evolution to any of the nucleic acid sequences of the present application are also within the scope of the present application.

In certain embodiments, substitutions, insertions, or deletions may be made in one or more of the complementarity determining regions or framework regions of the single-domain antibodies described herein, so long as such changes do not substantially reduce the ability of the antibody to bind antigen. For example, conservative changes (e.g., conservative substitutions, well known to those of skill in the art, conservative substitutions of amino acids that do not alter the properties and functions of the protein) can be made to the complementarity determining region and/or the framework region without substantially reducing binding affinity. For example, such changes may be outside the antigen-contacting residues in the complementarity determining regions.

The present application also provides a biomaterial related to the single-domain antibody BCMA-VHH-54, which can be any one of the following:

C1) a nucleic acid molecule encoding the heavy chain variable region of the single-domain antibody BCMA-VHH-54;
C2) an expression cassette including the nucleic acid molecule of C1);
C3) a recombinant vector including the nucleic acid molecule of C1) or a recombinant vector including the expression cassette of C2);

C4) a recombinant host cell including the nucleic acid molecule of C1), or including the expression cassette of C2), or including the recombinant vector of C3).

Wherein the recombinant host cell of C4) expresses the single-domain antibody BCMA-VHH-54. In the above biomaterial, the nucleic acid molecule may be any one of the following:

D1) a DNA molecule having a nucleotide sequence or a coding sequence of SEQ ID No.2;
D2) a DNA molecule having 75% or more identity to the nucleotide sequence defined in D1) and having the same function.

The DNA molecule shown in SEQ ID No.2 encodes the single-domain antibody BCMA-VHH-54 with the amino acid sequence SEQ ID No.1.

The 75% or more identity may be 80%, 85%, 90% or 95%, or more identity.

As used herein, identity refers to the identity of an amino acid sequence or a nucleotide sequence. Amino acid sequence identity may be determined using homology search sites on the Internet, such as the BLAST page of the NCBI homepage. For example, the value of identity (%) can be obtained in advanced BLAST 2.1 by using blastp as the program, setting the Expect value to 10, setting all Filter to OFF, using BLOSUM62 as the Matrix, setting Gap existence cost, Per residue gap cost and Lambda ratio to 11, 1 and 0.85 respectively (default values) and performing a search to calculate the identity of an amino acid sequence.

As used herein, a host cell (also referred to as a recipient cell) refers to any type of cell that can be used to introduce a vector. The host cell can be a eukaryotic cell (e.g., HEK293, CHO, myeloma cells, and the like), or can be a prokaryotic cell (e.g., E. coli, and the like). The host cells are understood to refer not only to the particular recipient cell, but also to the progeny of such a cell, and such progeny may not necessarily be identical to the original parent cell, due to natural, accidental, or deliberate mutation and/or alteration, but are still included within the scope of the host cell. In one or more embodiments of the present application, the host cells are E. coli TG1 and/or HEK293 cells.

As used herein, a recombinant vector refers to a recombinant DNA molecule constructed by linking an exogenous gene of interest to a vector in vitro, and can be constructed in any suitable manner as long as the constructed recombinant vector can carry the exogenous gene of interest into a recipient cell and provide the recipient cell with the ability to replicate, integrate, amplify and/or express the exogenous gene of interest. In one or more embodiments of the present application, the recombinant vector is pcDNA3.1-BCMA-VHH-54-hFc.

The recombinant vector pcDNA3.1-BCMA-VHH-54-hFc is constructed to facilitate purification by adding a human IgG1Fc gene (hFc gene, nucleotide sequence as shown in SEQ ID No.3) to the 3' end of the encoding gene of the single-domain antibody BCMA-VHH-54 (SEQ ID No.2) to obtain the BCMA-VHH-54-hFc fusion gene, then adding a *Bam*HI restriction site before the start codon at the 5' end of the fusion gene and an *Eco*RI restriction site after the stop codon at the 3' end, and cloning it into the *Bam*HI and *Eco*RI recognition sites of the eukaryotic expression vector pcDNA3.1, thereby obtaining the recombinant eukaryotic expression vector pcDNA3.1-BCMA-VHH-54-hFc that expresses the single-domain antibody BCMA-VHH-54 with a C-terminal fused hFc gene. The recombinant eukaryotic expression vector pcDNA3.1-BCMA-VHH-54-hFc contains the encoding gene of the single-domain antibody BCMA-VHH-54 as shown in SEQ ID No.2.

The nucleotide sequence (696 bp) of the human IgG1Fc gene is shown in SEQ ID No.3, and the encoded amino acid sequence is shown in SEQ ID No.4.

The present application also provides the use of the single-domain antibody BCMA-VHH-54 and/or the biomaterial in any of the following applications:

E1) use in the preparation of a drug for preventing or treating tumors, or use in preventing or treating tumors;
E2) use in the preparation of a drug for preventing or treating a BCMA-target-related disease, or use in preventing or treating a BCMA-target-related disease;
E3) use in the preparation of a product for screening for, diagnosing, or aiding in the diagnosis of a BCMA-target-related disease, or use in screening for, diagnosing, or aiding in the diagnosis of a BCMA-target-related disease;
E4) use in detecting BCMA protein or the preparation of a product for detecting BCMA protein; E5) use in the preparation of a product for binding to BCMA protein;
E6) use in mediating drug-specific recognition of tumors expressing BCMA antigen or in the preparation of a product for mediating drug-specific recognition of tumors expressing BCMA antigen;
E7) use in the in vivo imaging of BCMA protein or the preparation of a product for in vivo imaging of BCMA protein;
E8) use in the preparation of CAR cells targeting BCMA.

As used herein, the BCMA-target-related disease can be a BCMA-positive cancer (e.g., multiple myeloma).

Herein, the BCMA-positive cancer may include multiple myeloma (MM), chronic myelocytic leukemia (CML), acute B-lymphoblastic leukemia (ALL), non-Hodgkin lymphoma (NHL), and Hodgkin lymphoma, but is not limited thereto.

The product described in E6) that mediates drug-specific recognition of tumors expressing BCMA antigen may be a drug delivery system specifically targeting BCMA protein, which contains the single-domain antibody BCMA-VHH-54.

The drug delivery system can be a liposomal drug delivery system, a polymeric micellar drug delivery system, a polymeric disc drug delivery system, or a nanoparticle drug delivery system. The drug delivery system can be used for targeted delivery and/or site-directed release of drugs. The present application also provides a kit containing the single-domain antibody BCMA-VHH-54, which can have any of the following uses:

F1) in detecting BCMA protein;

F2) in screening for, diagnosing, or aiding in the diagnosis of a BCMA-target-related disease;

F3) in in vivo imaging of BCMA protein.

The kit may be a chemiluminescent immunoassay kit, an enzyme-linked immunoassay kit, a colloidal gold immunoassay kit, or a fluorescent immunoassay kit, but is not limited thereto. Further, the kit may also include reagents required for immunodetection, such as, but not limited to, labeled antibodies or antigens, magnetic particles, blocking solution, diluent, washing solution, color developing solution, stop solution, etc.

The various reagent components of the kit may be present in separate containers or may be pre-combined, in whole or in part, into a reagent mixture.

The components of the kit may be provided in the form of a solution, for example, an aqueous solution. In the case of being in an aqueous solution state, the concentration or content of these components can be conveniently determined by a person skilled in the art according to different requirements. For example, for storage purposes, the concentration of a component may be present at a higher concentration, and when in working condition or in use, the concentration may be reduced to a working concentration by diluting the aforementioned higher concentration solution. The kit may further include, for example, a buffering agent, preservative, or protein stabilizer. The kit may further include components necessary for the detection of the detectable label, such as an enzyme or a substrate. The kit may also contain a control sample or a series of control samples, which can be assayed and compared with the test sample. The kit may include written instructions on or in the kit container. The written instructions describe how to use the reagents contained in the kit.

As used herein, detecting BCMA protein can be detecting the presence of BCMA protein in a test sample and/or detecting the amount of BCMA protein in a test sample.

The sample to be tested may be a cell or tissue sample.

Products for detecting BCMA protein described herein include products for detecting antigen-antibody binding using enzyme-linked immunosorbent assay, immunofluorescence assay, radioimmunoassay, luminescent immunoassay, colloidal gold immunochromatography, agglutination method, or immunoturbidimetric method, etc.

The products described herein can be reagents, kits, chips, or test strips.

The present application also provides a pharmaceutical composition that can contain the single-domain antibody BCMA-VHH-54.

Further, the pharmaceutical composition may also contain a pharmaceutically acceptable carrier. The pharmaceutically acceptable carrier can be a diluent, excipient, filler, binder, wetting agent, disintegrant, absorption enhancer, adsorptive carrier, surfactant, or lubricant.

The pharmaceutical composition may have at least one of the following uses:

M1) for preventing or treating tumors;

M2) for preventing or treating a BCMA-target-related disease;

M3) for mediating drug-specific recognition of tumors expressing BCMA antigen.

The dosage form of the pharmaceutical composition may include inhalation preparations, oral preparations, and injection preparations, but is not limited thereto. The inhalation preparation may be an inhalation aerosol, inhalation powder, inhalation spray, inhalation liquid preparation, inhalation nebulizer, lyophilized powder for inhalation, or nasal spray. The oral preparation may be a tablet, powder, capsule, granule, pill, powder, ointment, solid beverage, or oral liquid. The injection preparation may be an injectable solution or powder for injection.

In order to prepare the pharmaceutical composition into injection preparations, such as solutions, emulsions, lyophilized powders for injection, and suspensions, all diluents (carriers) commonly used in the art can be used, for example, water, saline, phosphate-buffered saline, ethanol, polyethylene glycol, 1,3-propanediol, ethoxylated isostearyl alcohol, polyoxylated isostearyl alcohol, polyoxyethylene sorbitol fatty acid esters, and the like. In addition, to prepare an isotonic injection, a suitable amount of carriers such as sodium chloride, glucose, or glycerin can be added to the injection preparation; in addition, conventional carriers such as cosolvents, buffers, and pH adjusters can also be added.

The pharmaceutical composition can be prepared according to methods well-known and commonly practiced in the art. See e.g., Remington: The Science and Practice of Pharmacy, 22nd ed., Loyd V. Allen, et al., eds., Pharmaceutical Press (2012). The pharmaceutical composition is preferably manufactured under GMP or cGMP conditions.

The present application also provides a preparation method for the single-domain antibody BCMA-VHH-54, which method may include: constructing a recombinant expression vector containing a nucleic acid molecule described herein (a nucleic acid molecule encoding the heavy chain variable region of the single-domain antibody BCMA-VHH-54); introducing the recombinant expression vector into a host cell to obtain a recombinant cell expressing the single-domain antibody; culturing the recombinant cell, isolating and purifying to obtain the single-domain antibody.

Further, the nucleic acid molecule encoding the heavy chain variable region of the single-domain antibody BCMA-VHH-54 can be any of the following:

D1) a DNA molecule having a nucleotide sequence or a coding sequence of SEQ ID No.2;
D2) a DNA molecule having 75% or more identity to the nucleotide sequence defined in D1) and having the same function.

Further, the host cell may be a HEK293 cell.

Further, the introduction may be by transforming the host cell with a vector carrying the single-domain antibody gene of the present application by any known transfection method, such as calcium phosphate co-precipitation, liposome-mediated, electroporation, or viral vector method, etc.

The present application also provides a method for diagnosing or aiding in the diagnosis of the BCMA-target-related disease, which method may include: isolating and obtaining a sample from a subject, then using the single-domain antibody BCMA-VHH-54 to detect the content of BCMA protein in the sample, and performing diagnosis or auxiliary diagnosis of a BCMA-target-related disease according to the content of the BCMA protein.

Further, the method may further include: a step of comparing the content of BCMA protein in the detection result with a reference value (the content of BCMA protein for a disease diagnostic standard). The reference value can be a level of a BCMA protein in a sample from a subject (e.g., a healthy control) that is known not to have a BCMA-target-related disease (also referred to as a "negative reference value"). The result of the comparison may indicate whether the subject has or is at risk of having a disease associated with a BCMA target, or whether the patient's condition has progressed and/or is recovering from the treatment.

The present application also provides a method for screening for the BCMA-target-related disease, which method may include: isolating and obtaining a sample from a subject, then detecting the content of BCMA protein in the sample using the single-domain antibody BCMA-VHH-54, and screening for a BCMA-target-related disease according to the content of the BCMA protein (compared with the content of BCMA protein of a disease screening standard).

Further, the method may further include: a step of comparing the content of BCMA protein in the detection result with a reference value (the content of BCMA protein in a disease screening standard). The reference value can be a level of a BCMA protein in a sample from a subject (e.g., a healthy control) that is known not to have a BCMA-target-related disease (also referred to as a "negative reference value"). The results of the comparison may be indicative of a broad population suitable for the therapeutic products of the present application.

The present application also provides a method for preventing or treating a BCMA-target-related disease, which method can include administering the single-domain antibody BCMA-VHH-54 or a pharmaceutical composition described herein to a subject having a BCMA-target-related disease. In the above methods, the BCMA-target-related disease can be a BCMA-target-related tumor.

In the above methods, the BCMA-target-related tumor can be a BCMA-positive cancer.

In the above methods, the BCMA-positive cancer can include, but is not limited to, multiple myeloma, chronic lymphocytic leukemia, acute B-lymphoblastic leukemia, non-Hodgkin lymphoma, and Hodgkin lymphoma.

Further, the amount administered may be a therapeutically effective amount. The therapeutically effective amount may refer to (i) the treatment or prevention of the particular disease, condition, or disorder; (ii) alleviating, ameliorating, or eliminating one or more symptoms of a particular disease, condition, or disorder; or (iii) an amount of the drug that prevents or delays the onset of one or more symptoms of the particular disease, condition, or disorder described herein. A therapeutically effective amount can be determined by testing in known in vitro or in vivo (e.g., animal model) systems.

Further, such administration includes, but is not limited to, intramuscular injection, subcutaneous injection, intradermal injection, transdermal injection, intravenous injection, intra-arterial injection, intraperitoneal injection, intraperitoneal injection, intrathecal injection, microneedle injection, intratumoral injection, mucosal administration, oral administration, oro-nasal spray, aerosol inhalation, in vivo implant, and in vitro carrier device administration.

Preferred modes of administration (routes of administration) are parenteral (e.g., intravenous, intramuscular, intraperitoneal, intrasternal, subcutaneous, and intraarticular). The skilled artisan will appreciate that the route of administration will vary according to the intended purpose. In certain embodiments, the Nanobody, bispecific antibody or multispecific antibody, isolated nucleic acid molecule, recombinant vector, recombinant host cell, antibody conjugate, or pharmaceutical composition of the disclosure can be administered by injection or continuous infusion, including, but not limited to, intravenous, intraperitoneal, intradermal, subcutaneous, intramuscular, intraocular, and intraportal.

The present application also provides a method for detecting BCMA protein in vitro, which method may include detecting BCMA protein using the single-domain antibody BCMA-VHH-54 or the kit described herein.

The present application also provides an antibody-drug conjugate including an antibody moiety and a conjugate moiety, wherein the antibody moiety can include the single-domain antibody BCMA-VHH-54.

Further, the conjugate moiety may include a detectable label, a chemical drug, or a cytotoxin.

The antibody moiety and the conjugate moiety may be covalently linked either directly or through a linker.

The detectable label can be selected from an enzyme (e.g., horseradish peroxidase or alkaline phosphatase), a chemiluminescent reagent (e.g., acridinium esters, luminol and its derivatives, or ruthenium derivatives), a fluorescent dye (e.g., fluorescein or a fluorescent protein), a radionuclide, or biotin.

The chemical drug may be an agent that kills tumor cells.

The cytotoxin can be a tubulin inhibitor (e.g., maytansine, auristatin), an inhibitor of DNA synthesis (e.g., calicheamicin), or an inhibitor of RNA synthesis (e.g., amanitin).

The purposes of the uses and methods described herein may be disease diagnosis purposes, disease prognosis purposes and/or disease treatment purposes, and they may also be non-disease diagnosis purposes, non-disease prognosis purposes and non-disease treatment purposes; their direct purpose may be to obtain information of disease diagnosis results, disease prognosis results, and/or intermediate results of disease treatment results, and their direct purpose may be non-disease diagnosis purposes, non-disease prognosis purposes, and/or non-disease treatment purposes.

The subject described herein can be a human or a non-human animal. In certain embodiments, the subject has a BCMA-target-related disease.

The non-human animal described herein may be a non-human mammal.

The non-human mammal described herein may be any one of, but not limited to, a mouse, rat, guinea pig, hamster, pig, dog, sheep, monkey, rabbit, cat, cow, or horse.

BCMA (CD269, TNFRSF17), a type III transmembrane glycoprotein, belongs to the human tumor necrosis factor receptor (TNFR) superfamily and plays a major role in the maturation and differentiation of B cells into plasma cells. BAFF and APRIL are two B-cell activation and expansion-inducing ligands that bind to BCMA and together regulate B-cell function. BCMA is mainly expressed in mature B cells, and its expression gradually increases with the maturation of B cells, and is selectively overexpressed in plasma cells during malignant transformation, which promotes tumor cell growth, survival, and the development of drug resistance. BCMA is expressed on tumor cells of multiple myeloma, acute B-lymphoblastic leukemia, non-Hodgkin lymphoma, and Hodgkin lymphoma, among others.

**Advantageous Effects**

The single-domain antibody BCMA-VHH-54 of the present application has a high affinity, can well specifically target BCMA-positive cells (cells expressing BCMA protein), and can be successfully used in detecting BCMA expression in bone marrow cells of MM patients. The single-domain antibody of the present application can be expressed and produced in prokaryotic cells, yeast cells, eukaryotic cells, and any recombinant system, and can be prepared into a specific antibody drug clinically used for preventing and treating BCMA-target-related diseases (multiple myeloma, acute B-lymphoblastic leukemia, non-Hodgkin lymphoma, and Hodgkin lymphoma); or a BCMA protein detection kit, etc. The single-domain antibody has a stable structure, a small molecular size, is easily recombinantly expressed, and has a low production cost. It can be used alone or as a drug delivery system to carry relevant drugs, and has very wide prospects and important significance in fields such as drug application and clinical diagnosis.

**Brief Description of the Drawings**

FIG. 1 is a research flow chart of the present application.

FIG. 2 shows the results of the detection of BCMA expression on different tumor cell lines using FITC-labeled BCMA-VHH-54 antibody. Wherein Isotype represents the control group, and BCMA represents the FITC-BCMA-VHH-54-hFc antibody.

FIG. 3 shows the results of the detection of BCMA expression on bone marrow cells of MM patients using FITC-labeled BCMA-VHH-54 antibody. FMOC is the control group, and BCMA-FITC represents the FITC-labeled BCMA-VHH-54 antibody.

**Detailed Description of the Invention**

The present application is described in further detail below in conjunction with specific embodiments, which are provided only to illustrate the present application and not to limit the scope of the present application. The following examples can serve as a guide for further improvements by those of ordinary skill in the art, and do not constitute any limitation to the present application.

The experimental methods in the following examples, unless otherwise specified, are conventional methods, and are carried out according to the techniques or conditions described in the literature in the field or according to the product instructions. The materials, reagents, etc., used in the following examples are commercially available unless otherwise specified.

The pComb3XSS plasmid in the following examples is a product of Beijing Zoman Biotechnology Co., Ltd., Cat. No.: ZK129.

The helper phage M13K07 in the following examples is a product of Chengdu Renyu Biotechnology Co., Ltd., Cat. No.: A001-1.

The K562 cells (BCMA-negative cells) in the following examples are products of Nanjing Kebai Biotechnology Co., Ltd., Cat. No.: CBP60529; the KG-1a cells (BCMA-negative cells) are products of Nanjing Kebai Biotechnology Co., Ltd., Cat. No.: CBP61455; the RPMI-8226 (BCMA-positive cells) are products of Nanjing Kebai Biotechnology Co., Ltd., Cat. No.: CBP60244; the Daudi (BCMA-positive cells) are products of Nanjing Kebai Biotechnology Co., Ltd., Cat. No.: CBP60262; the MM1.S cells (BCMA-positive cells) are products of Biobw Co., Ltd., Cat. No. bio-129535.

In the following examples, the bone marrow cells of MM patients were obtained from Zhejiang Provincial People's Hospital.

In the following examples, the preparation method for BCMA-positive K562-BCMA cells is to insert BCMA between the *Bam*HI and *Eco*RI sites of pcDNA3.1, transfect K562 cells with the prepared recombinant vector, and obtain BCMA-positive K562-BCMA cells.

The vector pcDNA3.1 in the following examples is a product of Changsha Abiowell Biotechnology Co., Ltd., Cat. No.: HG-VPI0001.

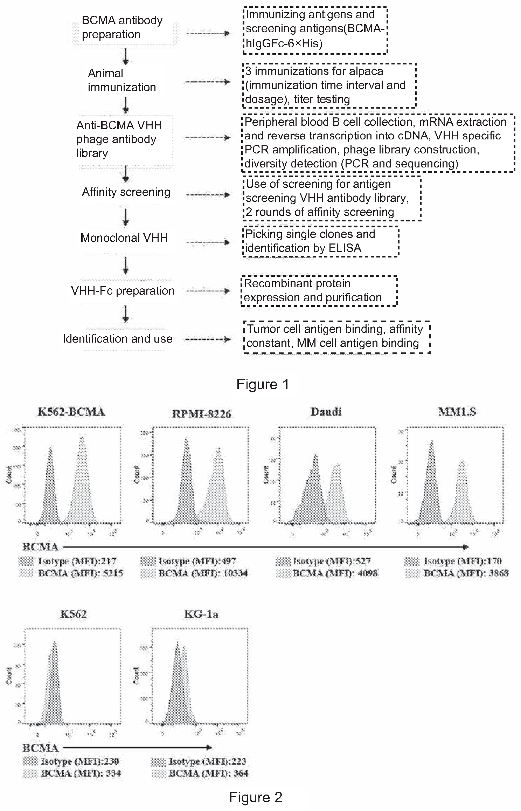

The research flow chart of the present application is shown in FIG. 1.

Example 1: Screening and preparation of anti-BCMA single-domain antibodies

In the embodiment, alpacas were immunized with BCMA-FC recombinant protein to obtain single-domain antibody genes, and a single-domain antibody expression library was constructed.

Then, high-affinity anti-BCMA single-domain antibodies were screened from the single-domain antibody expression library by phage display screening technology. The specific steps were as follows:

1. Animal immunization

1.1. Obtaining the antigen

The BCMA recombinant protein used as the antigen is shown below:

BCMA immunoantigen: Human BCMA Llama IgG2b FC (BCA-H5259) protein is a product of Shanghai Bointron Biotechnology Co., Ltd.
BCMA Screening Antigen: Human BCMA-6xHis (BCA-H522Y) protein is a product of Shanghai Bointron Biotechnology Co., Ltd.

1.2. Immunization of alpaca

Alpaca (2-3 years old, body weight about 50 kg) was immunized three times with BCMA antigen Human BCMA Llama IgG2b FC by subcutaneous injection of 0.5 mg Human BCMA Llama IgG2b FC each time, with a two-week interval between immunizations. Prior to the next immunization, 5 ml of blood was drawn, and plasma was separated to detect the antibody titer after the previous immunization. The titer of the third immunization was determined one week after the injection.

The titers of the antibodies in the serum were determined by the indirect ELISA method as follows:

A. The antigen was diluted to 5 $\mu$g/mL with PBS and coated at 100 $\mu$L/well overnight at 4°C;
B. The coating solution was discarded, and the plates were washed three times with PBST. Then, 300 $\mu$L of 5% skim milk was added per well for blocking at 37°C for 1 h;

C. The plates were washed three times with PBST, 100 μL/well of diluted serum (starting from a 1:2000 dilution) was added, followed by incubation at 37°C for 45 min;

D.The plates were washed five times with PBST, and horseradish peroxidase-labeled goat anti-alpaca secondary antibody (diluted 1:10,000 in PBS) was added at 100 μL/well and incubated at 37°C for 45 min;

E. The plates were washed five times with PBST. TMB developing solution was added to develop color, 100 μL/well, 37°C, 5 min, stop solution was added to stop the reaction, 50 μL/well, and the optical density was measured at 450 nm. Determination criteria for positive wells: the OD450 reading of the immunized serum sample was greater than that of the unimmunized serum, and the reading was greater than 0.5.

1.3. Immunization results

The results of the serum titer test are shown in Table 1, with the positive wells in bold font. After three immunizations, the serum titer of B alpaca reached 64K, i.e., the antibody level reached 64K.

Table 1. Test results of serum titer

| Dilution | Second immunization | Third immunization | Second immunization | Third immunization |
|---|---|---|---|---|
| | A alpaca | | B alpaca | |
| 1:2K | 0.0609 | 0.1573 | 1.2114 | **3.0033** |
| 1:4K | 0.0538 | 0.1010 | 0.8225 | **2.4910** |
| 1:8K | 0.0525 | 0.0810 | 0.5097 | **2.2286** |
| 1:16K | 0.0464 | 0.0937 | 0.3469 | **1.5065** |
| 1:32K | 0.0475 | 0.0624 | 0.2027 | **0.8956** |
| 1:64K | 0.0466 | 0.0514 | 0.1185 | **0.5198** |
| 1: 128K | 0.0463 | 0.0556 | 0.1062 | 0.2561 |
| BLANK | 0.0578 | 0.0479 | 0.0442 | 0.0443 |

As shown in Table 1, after the third immunization, the antibody level in alpaca reached 64K. The Ag coating was coated with the antigen Human BCMA Protein His tag.

2. Phage library construction

After successful immunization, peripheral blood mononuclear cells (PBMC) of the above-mentioned B alpaca were collected for phage library construction: that is, the anti-BCMA VHH phage antibody library.

2.1. RNA extraction and reverse transcription

Alpaca peripheral blood mononuclear cells (referred to as alpaca PBMC) were isolated by density gradient centrifugation and dissolved in Trizol for storage. Total RNA was extracted from alpaca PBMC, and the resulting total RNA was reverse transcribed into cDNA using the Takara reverse transcription kit. The specific steps were as follows:

A: Peripheral blood lymphocyte isolation

50 mL of peripheral blood was collected from B alpaca after the third immunization, and PBMCs were then isolated according to the manufacturer's instructions for the lymphocyte separation solution.

B: RNA extraction

Total RNA was extracted with RNAiso Plus reagent.

C: Determination of RNA purity by gel electrophoresis

The purity of RNA was detected by electrophoresis with 1 μg RNA. The results showed that the purity of RNA was good.

D: Reverse transcription

See PrimeScript™ II 1st Strand cDNA Synthesis Kit (Takara, Cat. No.:6210A) instructions, and a total of 5 μg RNA was reverse-transcribed.

2.2. PCR amplification of the VHH target fragment of BCMA antibody

The obtained cDNA stock solution was mixed in equal proportions and then diluted five times, and 5.0 μL was selected as the optimal amplification concentration. Approximately 750 bp of the VHH fragment was recovered by gel cutting after the first round of nested PCR, and the VHH fragment was purified using a DNA product purification kit after the second round of nested PCR. 2.3. Enzymatic cleavage and ligation

1) Using pComb3XSS as the phage plasmid vector, the pComb3XSS vector and VHH PCR product (i.e., the VHH target fragment) were digested with restriction enzyme SfiI, respectively, and incubated overnight at 50°C.
2) The digested pComb3XSS vector and the PCR product of VHH were purified using the DNA Recovery Purification Kit to obtain a total of 3.1 μg of ligation product, which was stored at 4°C.
3) The digested pComb3XSS vector and the target fragment of VHH were ligated and recovered, and a total of 3.1 μg of ligation product was obtained. The ligation molar ratio was Vector : VHH = 1:3.

2.4. Bacterial library construction

A total of 10 electrotransformations were performed. Immediately after electroshock, 1 mL of 2YT medium (preheated at 37°C) was added to the electroporation cuvette for resuscitation. The electroshock products were aspirated, and the electroporation cuvette was washed with 2YT medium. A total of 100 ml of recovery mixture was obtained and incubated at 37°C with shaking at 180 rpm for 45 min. 100 μL of the mixture was serially diluted to $10^{-3}$ and $10^{-4}$ to determine the number of library transformants, and plated on 90 mm plates. The rest were centrifuged, resuspended by adding 8 mL of 2YT medium, and plated on 8 pieces of 200 mm plates. The following day, a total of 105 clones were obtained on the plates for determining the number of library transformants. Forty-eight clones were randomly picked from the titer plates to determine the number of library transformants and identified, and the results showed that the insertion rate was 100%. According to the analysis results of the number of library transformants, library insertion rate, and diversity sequencing, the capacity of the constructed library was $1.05 \times 10^9$ ($105 \times 1000 \times 10^4 \times 1 \times 1$). The library had good capacity and diversity, which could be used for the next screening.

2.5. Phage library preparation

(1) The bacterial library was inoculated into 2 x 300 mL of 2YT+A+G medium (Amp:100 ug/ml, Glu:1%) with an initial OD600 = 0.1-0.2, cultured at 37°C, 230 rpm until OD600 ≥ 0.8.
(2) Helper phage M13KO7 was added based on the OD600 value (Helper phage : bacteria = 20:1).
(3) After helper phage M13KO7 was added, the culture was mixed gently and allowed to stand at 37°C for 30 min.
(4) The culture was shaken gently at 180 rpm at 37°C for 30 min.
(5) The culture was centrifuged at 5000 rpm for 10 min, the supernatant was discarded completely, and an equal volume of 2YT+A+K (Amp:100 μg/ml, Kan:50 μg/ml) medium was used to resuspend the pellet, and the culture was cultured overnight at 30°C with 220 rpm.
(6) The overnight culture was centrifuged at 10000 rpm for 20 min at 4°C, the supernatant was collected, and the pellet was discarded. The centrifuge tube was replaced, centrifuged at 10000 rpm for 20 min at 4°C, and the supernatant was collected.
(7) PEG8000/NaCl was added at a ratio of 1/5 of the supernatant volume, mixed well, and precipitated in an ice bath for more than 2 h.
(8) The culture was centrifuged at 10000 rpm for 20 min at 4°C, the supernatant was discarded, and a brief centrifugation was performed to remove residual supernatant completely. The precipitate was suspended with 1 mL of 1x PBS, and 1/5 volume of PEG8000/NaCl was added for secondary precipitation for 1 h.
(9) The culture was centrifuged at 12000 rpm for 10 min at 4°C, the supernatant was discarded, and a brief centrifugation was performed to remove residual supernatant completely. Depending on the amount of the pellet, 1x PBS was added to resuspend the pellet.
(10) 100% glycerol was added to a final concentration of 50%, the mixture was mixed well, dispensed into 1.5 mL EP tubes, and stored at -80°C.

(11) 10 μL of the phage library was diluted with 2YT by gradient dilution, 10 μl from the $10^{-8}$ and $10^{-9}$ tubes was taken and added to 90 μL of TG1 bacterial solution, and mixed gently. After standing at 37°C for 15 min, the mixture was plated on Amp-resistant plates respectively and cultured overnight.

(12) The next day, a total of 540 clones were obtained on the $10^{-9}$ titer plates, and the phage library titer was 5.40 x $10^{13}$ cfu/mL (540 x $10^9$ x 100).

3. Affinity screening

3.1 The phage library was subjected to 2 rounds of affinity screening using the screening antigen (BCMA-6xHis), including titer detection, amplification of phage eluate, and purification of phage.

1) The target molecule BCMA-6xHis antigen was diluted with carbonate buffer (pH 9.6) to a final concentration of 5 μg/mL, 100 μL/well was added into microplate wells, 8 wells coated per target molecule (4 wells coated for the second round of screening, 2 wells each for the third and fourth rounds), coating was performed overnight at 4°C;

2) The coating solution was discarded, and the plates were washed three times with PBS, 300 μL of 3% OVA-PBS blocking solution was added to each well. Blocking was performed at 37°C for 1 h;

3) The plates were washed three times with PBS, 100 μL of phage library was added, and incubation was performed at 37°C for 1 h;

4) Unbound phage was aspirated, the plates were washed six times with PBST and two times with PBS;

5) 100 μL Gly-HCl elution solution was added, and incubation was performed at 37°C for 8 min to elute specifically bound phage; the eluent was transferred to a 1.5 mL sterile centrifuge tube and was quickly neutralized with 10 μL Tris-HCl neutralization buffer;

6) 10 μL was taken for gradient dilution, and the titer was determined. The panning recovery rate was calculated. The remaining eluates were mixed, amplified, and purified for the next round of affinity panning with modified screening conditions.

3.2 Amplification of the library after panning

1) The panning eluate was mixed with 20 mL of E. coli TG1 culture at early-log phase, incubated at 37°C for 30 min without shaking. 1 ml of 20% glucose was added, and the mixture was incubated at 220 rpm for 30 min. M13K07 helper phage was added at a ratio of cell : phage = 1:20 along with 4 μl of Amp+, then incubated statically at 37°C for 30 min. 2 ml of 2YT liquid medium was supplemented, and the culture was incubated with shaking at 220 rpm for 30 min;

2) The culture was dispensed into centrifuge tubes and centrifuged at 4°C, 5000 r/min for 10 min. The cell pellets were resuspended in 50 mL of 2x YT-AK liquid medium, and the suspension was incubated overnight at 30°C with shaking at 250 r/min;

3) The overnight culture was centrifuged at 4°C, 10000 r/min for 20 min. The supernatant was transferred to a new centrifuge tube, where 1/5 volume of PEG-NaCl was added, mixed thoroughly, and placed at 4°C for ≥2 h;

4) The mixture was centrifuged at 4°C, 10000 r/min for 20 min. After removing the supernatant, the pellet was resuspended in 1 mL PBS, mixed with 1/5 volume of PEG/NaCl, and incubated at 4°C for ≥1 h;

5) The mixture was centrifuged at 4°C, 12000 r/min for 2 min. The supernatant was discarded, and the pellet was suspended in 200 μL PBS to obtain the amplification product. The titer was determined, and the product was either used for the next panning round or analysis.

3.3 Identification and analysis of specific phage clones

1) From the plates with the titer of panning eluate, 480 clones were randomly picked from each second-round titer plate using sterilized toothpicks, inoculated into 300 μl of 2x YT-A, and incubated with shaking at 230 rpm at 37°C for 8 h.

2) 100 μL of the aforementioned culture was taken, mixed with M13K07 phage at a ratio of cell : phage = 1:20, at 37°C, it was allowed to stand for 15 min, incubated with shaking at 220 rpm for 45 min.

3) 300 μL of 2x YT-AK was added, and the mixture was incubated overnight at 30°C with vigorous shaking.

4) The next day, the culture was centrifuged at 12,000 rpm for 2 min, and the supernatant was collected for monoclonal ELISA identification.

3.4 Identification of Positive Phage Clones

1) The target molecule hBCMA-his antigen was diluted with carbonate buffer at pH 9.6 to a final concentration of 2 μg/mL, 100 μL/well was added to the microplate wells respectively, and coated overnight at 4°C;
2) The coating solution was discarded, the plates were washed three times with PBST, 300 μL of 5% skim milk was added into each well, and blocked at 37°C for 1 h;
3) The plates were washed once with PBST, and 50 μL of phage culture supernatant and 50 μL of 5% skim milk were added into each well, and the mixture was incubated at 37°C for 1 h;
4) The plates were washed five times with PBST, and horseradish peroxidase-labeled anti-M13 antibody (diluted 1:10,000 with PBS) was added, 100 μL/well, and the mixture was incubated at 37°C for 1 h;
5) The plates were washed six times with PBST. The TMB developing solution was added to develop color, 100 μL/well, 37°C, 7 min, stop solution was added to stop the reaction, 50 μL/well, and the optical density was measured at 450 nm.
6) hBCMA antigen affinity panning: the Gly-HCl acid elution method was used for screening, and the enrichment degree during the screening process is shown in the following table.

Table 2. Recovery amount of acid elution screening using hBCMA antigen as the target molecule

| Rounds | Antigen coating concentration (μg/mL) | Library input amount (cfu) | Recovery amount (cfu) | Recovery rate | Enrichment factor |
|--------|---------------------------------------|----------------------------|------------------------|---------------|-------------------|
| 1 | 5 | $2 \times 10^{11}$ | $1.70 \times 10^{5}$ | $8.5 \times 10^{-7}$ | |
| 2 | 2 | $1 \times 10^{11}$ | $1.54 \times 10^{7}$ | $1.54 \times 10^{-4}$ | 181.18 |

4. BCMA-VHH monoclonal ELISA identification

The hBCMA antigen was used as the target molecule, and specific phages were eluted by Gly-HCl acid elution. 480 clones (numbered NB405-ANTI-Human-1-96, NB405-ANTI-HBCMA-97-480; 1-480 as second-round monoclonals) were randomly picked from the second-round titer determination plate of HBCMA using sterilized toothpicks. Positive clones were screened by indirect ELISA, and the optical density value was measured at 450 nm.

After two rounds of screening, a total of 480 clones were identified on the second-round titer plate, yielding 317 positive clones. Among them, clones 48, 342, and 423 were non-monoclonal. Clones 146, 150, 155, 321, 414, and 445 were retested as negative clones. Analysis of sequencing data from the above positive clones revealed 41 unique sequences based on differences in CDR3 sequences. Combining the results of positive clone ELISA screening, we selected clone 54 to express VHH-hIgG1Fc using HEK293 cells.

5. Preparation of anti-BCMA single-domain antibody

5.1 Structure and sequence of the anti-BCMA single-domain antibody

Positive monoclonals from step 4 were selected and sequenced to obtain antibody gene sequences. Anti-BCMA single-domain antibodies (BCMA-VHH) were prepared via genetic engineering, with one antibody named BCMA-VHH-54.

A single-domain antibody contains only the variable region (VHH) of a heavy chain antibody, consisting sequentially of framework region FR1, complementarity determining region CDR1, framework region FR2, complementarity determining region CDR2, framework region FR3, complementarity determining region CDR3, and framework region FR4. The specific sequence information is as follows:
The amino acid sequence of the single-domain antibody BCMA-VHH-54 is SEQ ID No.1. Positions 1-25 of SEQ ID No.1 are framework region FR1, positions 26-33 are complementarity determining region CDR1, positions 34-50 are framework region FR2, positions 51-58 are complementarity determining region CDR2, positions 59-96 are framework region FR3, positions 97-106 are complementarity determining region CDR3, and positions 107-117 are framework region FR4. The nucleotide sequence of the nucleic acid molecule (BCMA-VHH-54 gene) encoding the single-domain antibody BCMA-VHH-54 is shown in SEQ ID No.2.

Amino acid sequence of the single-domain antibody BCMA-VHH-54: 5'-QLQLVESGGGLVQPGGSLKLSCAA SGFTFKDYAMNWYRQAPGKEREWVALISSDGGT TEYEQSVKGRFTISRDNAKNTVYLQMNSVKPEDTAVYRCNKFG

AGSYNYWGQGTLVTVS S-3' (SEQ ID No.1). The three underlined segments are CDR1, CDR2, and CDR3, respectively.

Nucleotide sequence of the BCMA-VHH-54 gene:

5'-CAGTTGCAGCTCGTGGAGTCAGGTGGAGGCTTGGTGCAGCCTGGGGGGTCTCTGA
AACTCTCCTGTGCAGCCTCTGGATTCACCTTCAAAGACTATGCCATGAACTGGTACCGT
CAGGCTCCAGGGAAGGAGCGCGAGTGGGTCGCACTTATTAGTAGTGATGGTGGTACCA
CAGAGTATGAGCAGTCCGTGAAGGGGCGATTCACCATCTCCAGAGACAATGCCAAGA
ACACGGTGTATCTGCAAATGAACAGCGTGAAACCTGAGGACACGGCCGTGTATCGCTG
TAATAAGTTCGGAGCAGGATCCTATAACTACTGGGGCCAGGGGACCCTGGTCACCGTC
TCCAGC-3' (SEQ ID No.2).

The sequences of the complementarity determining regions are defined according to the Kabat numbering system.

The aforementioned single-domain antibody is a single-domain antibody that specifically binds to the BCMA protein.

5.2 Expression and purification of anti-BCMA single-domain antibodies

The eukaryotic expression vector pcDNA3.1 was used to express the anti-BCMA single-domain antibody (BCMA-VHH-54).

5.2.1 Construction of recombinant vectors

To facilitate purification, a human IgG1Fc gene (hFc gene; nucleotide sequence: SEQ ID No.3, encoded amino acid sequence: SEQ ID No.4) was added to the 3' end of the gene encoding the single-domain antibody BCMA-VHH-54 (SEQ ID No.2) to generate the BCMA-VHH-54-hFc fusion gene. A BamHI restriction site was then inserted before the start codon at the 5' end of the fusion gene, and an EcoRI restriction site was inserted after the stop codon at the 3' end. The fusion gene was cloned into the eukaryotic expression vector pcDNA3.1 between the BamHI and EcoRI recognition sites, resulting in the recombinant eukaryotic expression vector pcDNA3.1-BCMA-VHH-54-hFc, which expresses the BCMA-VHH-54 single-domain antibody with a C-terminally fused hFc gene.

5.2.2 Expression of anti-BCMA single-domain antibody

The eukaryotic expression vector pcDNA3.1-BCMA-VHH-54-hFc obtained in step 5.2.1 was transfected into HEK293 cells by conventional electroporation for expression.

5.2.3 Purification of anti-BCMA single-domain antibodies

Protein A had the activity of binding to the Fc fragment of IgG, so the anti-BCMA single-domain antibody expressed by HEK293 cells was purified using a Protein A column (conventional purification procedure).

Finally, the purified anti-BCMA single-domain antibody fused with the hFc gene (BCMA-VHH-54-hFc) was prepared.

Example 2: Functional characterization of anti-BCMA single-domain antibodies

1. Antigen-antibody affinity determination

Antibodies to be tested: the anti-BCMA single-domain antibody fused with the hFc gene (BCMA-VHH-54-hFc) prepared in step 5.2.3.

(1) The antibody to be tested, BCMA-VHH-54-hFe, was labeled with the Pierce™ NHS-Fluorescein Antibody Labeling Kit to obtain the FITC directly-labeled antibody FITC-BCMA-VHH-54-hFc for flow cytometry detection;

(2) BCMA-positive K562-BCMA cells were resuspended to a density of 2 x 10$^6$ cells/mL, 100 μL of which was taken and placed respectively in a 96-well V-bottom plate, centrifuged to discard the supernatant, and the cells were collected (K562 and KG-1a cells served as BCMA-negative cell controls);

(3) 40 μL of FITC-BCMA-VHH-54-hFc diluted in different gradients (see Table 3 for specific groups) was mixed with the above cells, and incubated at room temperature in the dark for 10 min;

Table 3. FITC-BCMA-VHH-54-hFc Antibody Affinity Test Groups

| | Antibody concentration (μg/mL) | | | | | | | |
|---|---|---|---|---|---|---|---|---|
| K562-BCMA | 5.7 | 2.85 | 1.425 | 0.7125 | 0.3563 | 0.1781 | 0.0891 | 0.0445 |
| Antibody dilution ratio | 1:100 | 1:200 | 1:400 | 1:800 | 1:1600 | 1:3200 | 1:6400 | 1:12800 |

(4) The cells were washed twice with 200 μL FACS buffer by centrifugation, resuspended with 200 μL FACS buffer, and immediately subjected to flow cytometry to detect the mean fluorescence intensity (MFI);
(5) The affinity was determined by calculating the equilibrium dissociation constant (KD) for the binding of the BCMA VHH antibody to target cells according to the formula. The smaller the KD value, the stronger the affinity.

$$1/(MFI\text{-}Con) = 1/Fmax + (KD/Fmax)(1/[BCMA\ IgG\ Fc]).$$

MFI-Con represents the relative mean fluorescence intensity, which is the value obtained by subtracting the background mean fluorescence intensity from the mean fluorescence intensity of the experimental group; BCMA IgG Fc represents the amount of antibody used in units of nM.
A standard curve was plotted with the reciprocal of the amount of antibody used as the abscissa and the reciprocal of the relative mean fluorescence intensity as the ordinate, and a linear regression equation was obtained. According to the linear regression equation, the intersection point between the standard curve and the ordinate is 1/Fmax, and KD is the slope of the straight line multiplied by Fmax.
The calculated equilibrium dissociation constants are shown in Table 4:

Table 4. Equilibrium dissociation constant (KD) of BCMA-VHH-54-hFc and K562-BCMA cells

| Single-domain antibody | Affinity constant KD (nM) | Linear regression equation | Phase Difference Coefficient $R^2$ for Linear Regression |
|---|---|---|---|
| BCMA-VHH-5 4 | 26.9 | y = 0.004x+0.0004 | 0.999 |

2. Specificity detection of the single-domain antibody BCMA-VHH-54

FITC-labeled BCMA-VHH-54 antibody (FITC-BCMA-VHH-54-hFc) was used to detect the expression of BCMA on different tumor cells.

(1) BCMA-negative cell lines (control group): K562 cells and KG-1a cells; BCMA-positive cell lines (experimental group): RPMI-8226, Daudi, MM1.S cells and the BCMA-overexpressing cell line K562-BCMA were thawed at 37°C, added into cell culture flasks, cultured at 37°C and 5% $CO_2$, and used for detection of the anti-BCMA single-domain antibody (FITC-BCMA-VHH-54-hFc) after two subcultures.
(2) The concentration of each cell type was adjusted to $2 \times 10^6$ cells/mL, 200 μL/well was added to a 96-well plate, and centrifuged at 1500 rpm for 5 min;
(3) The cells were washed with FACS buffer and centrifuged;
(4) 20 μL of diluted FITC-BCMA-VHH-54-hFc antibody (1:400) was mixed well and incubated at room temperature for 10 min;
(5) The antibody was washed off by centrifugation with FACS buffer, then resuspended in buffer and detected using a flow cytometer.

Flow cytometry results showed that the FITC-BCMA-VHH-54-hFc antibody was able to specifically bind to the BCMA antigen molecule expressed on tumor cells (FIG. 2).

3. Detection of BCMA expression in bone marrow cells of MM patients

Bone marrow cells from 2 patients with multiple myeloma (MM) were detected using FITC-labeled BCMA-VHH-54 antibody (FITC-BCMA-VHH-54-hFc), and the flow cytometry analysis steps were as follows:

(1) Cryopreserved bone marrow cells from 2 patients with multiple myeloma (MM) were resuscitated at 37°C.

(2) The cell density was adjusted to 2 x 10$^6$ cells/ml, 200 μL/well was added to a 96-well plate, and centrifuged at 1500 rpm for 5 min;

(3) The cells were washed with FACS buffer and centrifuged;

(4) 20 μL of diluted FITC-BCMA-VHH-54-hFc antibody (1:400) was mixed well and incubated at room temperature for 10 min;

(5) The antibody was washed off by centrifugation with FACS buffer, then resuspended in buffer and detected using a flow cytometer.

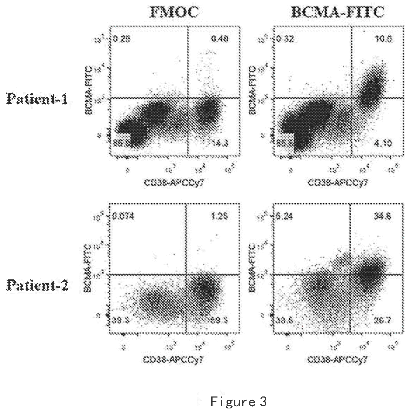

The results showed that the single-domain antibody BCMA-VHH-54 of the present application was able to specifically recognize BCMA expressed by MM tumor cells (FIG. 3).

The present application has been described in detail above. For those skilled in the art, the present application can be implemented within a wide range of equivalent parameters, concentrations, and conditions without departing from the spirit and scope of the present application and without the need for undue experimentation. Although specific examples of the present application have been given, it should be understood that further improvements can be made to the present application. In general, in accordance with the principles of the present application, the present application intends to cover any variations, uses, or improvements of the present application, including changes made by conventional techniques known in the art that depart from the scope disclosed in the present application.

## INDUSTRIAL APPLICABILITY

The single-domain antibody (BCMA-VHH-54) of the present application has high affinity, can well specifically target BCMA-positive cells (cells expressing BCMA protein), and can be successfully used in detecting BCMA expression in bone marrow cells of MM patients. The single-domain antibody of the present application can be expressed and produced in prokaryotic cells, yeast cells, eukaryotic cells, and any recombinant system, and can be prepared into a specific antibody drug clinically used for preventing and treating BCMA-target-related diseases (multiple myeloma, acute B-lymphoblastic leukemia, non-Hodgkin lymphoma, and Hodgkin lymphoma); or a BCMA protein detection kit, etc. The single-domain antibody has a stable structure, a small molecular size, is easily recombinantly expressed, and has a low production cost. It can be used alone or as a drug delivery system to carry relevant drugs, and has very wide prospects and important significance in fields such as drug application and clinical diagnosis.

## Claims

1. A single-domain antibody, **characterized in that** the single-domain antibody consists of a heavy chain variable region, and the heavy chain variable region comprises the amino acid sequences of complementarity determining region CDR1 from positions 26-33 of SEQ ID No.1, complementarity determining region CDR2 from positions 51-58 of SEQ ID No.1, and complementarity determining region CDR3 from positions 97-106 of SEQ ID No.1.

2. The single-domain antibody according to claim 1, **characterized in that** the amino acid sequence of the heavy chain variable region is SEQ ID No.1 or an amino acid sequence having at least 80% identity to SEQ ID No.1 and having the same function.

3. A biomaterial related to the single-domain antibody according to claim 1 or 2, **characterized in that** the biomaterial is any one of:

   C1) a nucleic acid molecule encoding the heavy chain variable region of the single-domain antibody according to claim 1 or 2;
   C2) an expression cassette comprising the nucleic acid molecule of C1);
   C3) a recombinant vector comprising the nucleic acid molecule of C1) or a recombinant vector comprising the expression cassette of C2);
   C4) a recombinant host cell comprising the nucleic acid molecule of C1), or comprising the expression cassette of C2), or comprising the recombinant vector of C3).

4. The biomaterial according to claim 3, **characterized in that** the nucleic acid molecule is any one of:

   D1) a DNA molecule having a nucleotide sequence or a coding sequence of SEQ ID No.2;
   D2) a DNA molecule having 75% or more identity to the nucleotide sequence defined in D1) and having the same function.

5. Use of the single-domain antibody according to claim 1 or 2 and/or the biomaterial according to claim 3 or 4 in any of the following:

E1) use in the preparation of a medicament for preventing or treating tumors, or use in preventing or treating tumors;
E2) use in the preparation of a medicament for preventing or treating a BCMA target-related disease, or use in preventing or treating a BCMA-target-related disease;
E3) use in the preparation of a product for screening for, diagnosing, or aiding in the diagnosis of a BCMA-target-related disease, or use in screening for, diagnosing, or aiding in the diagnosis of a BCMA-target-related disease;
E4) use in detecting a BCMA protein or in the preparation of a product for detecting a BCMA protein;
E5) use in the preparation of a product for binding to a BCMA protein;
E6) use in mediating drug-specific recognition of tumors expressing a BCMA antigen or in the preparation of a product for mediating drug-specific recognition of tumors expressing a BCMA antigen;
E7) use in *in vivo* imaging of a BCMA protein or in the preparation of a product for *in vivo* imaging of a BCMA protein;
E8) use in the preparation of CAR cells targeting BCMA.

6. The use according to claim 5, **characterized in that** the BCMA-target-related disease is a BCMA-positive cancer.

7. The use according to claim 6, **characterized in that** the BCMA-positive cancer comprises multiple myeloma, chronic lymphocytic leukemia, acute B-lymphoblastic leukemia, non-Hodgkin lymphoma, and Hodgkin lymphoma.

8. A kit, **characterized in that** the kit comprises the single-domain antibody according to claim 1 or 2, the kit has any of the following uses:

F1) in detceting BCMA protein;
F2) in screening for, diagnosing, or aiding in the diagnosis of a BCMA-target-related disease;
F3) in *in vivo* imaging of BCMA protein.

9. A pharmaceutical composition, **characterized in that** the pharmaceutical composition comprises the single-domain antibody of claim 1 or 2.

10. A method for preparing the single-domain antibody according to claim 1 or 2, **characterized in that** the method comprises: constructing a recombinant expression vector comprising the nucleic acid molecule of claim 3 or 4; introducing the recombinant expression vector into a host cell to obtain a recombinant cell expressing the single-domain antibody; culturing the recombinant cell, isolating and purifying to obtain the single-domain antibody.

11. A method for diagnosing or aiding in the diagnosis of a BCMA-target-related disease, **characterized in that** the method comprises: isolating and obtaining a sample from a subject, then detecting the content of a BCMA protein in the sample using the single-domain antibody of claim 1 or 2, and diagnosing or aiding in the diagnosis of the BCMA-target-related disease based on the content of the BCMA protein.

12. A method for screening for a BCMA-target-related disease, **characterized in that** the method comprises: isolating and obtaining a sample from a subject, then detecting the content of BCMA protein in the sample using the single-domain antibody of claim 1 or 2, and screening for the BCMA-target-related disease based on the content of the BCMA protein.

13. A method for preventing or treating a BCMA-target-related disease, **characterized in that** the method comprises administering the single-domain antibody of claim 1 or 2, or the pharmaceutical composition of claim 9, to a subject having a BCMA-target-related disease.

14. The method according to any one of claims 11 to 13, **characterized in that** the BCMA-target-related disease is a BCMA target-related tumor.

15. The method according to claim 14, **characterized in that** the BCMA-target-related tumor is a BCMA positive cancer.

**16.** The method according to claim 15, **characterized in that** the BCMA positive cancer comprises multiple myeloma, chronic lymphocytic leukemia, acute B-lymphoblastic leukemia, non-Hodgkin lymphoma, and Hodgkin lymphoma.

**17.** A method for detecting a BCMA protein *in vitro,* **characterized in that** the method comprises detecting the BCMA protein using the single-domain antibody of claim 1 or 2, or the kit of claim 8.

**18.** An antibody-drug conjugate, comprising an antibody moiety and a conjugate moiety, **characterized in that** the antibody moiety comprises the single-domain antibody of claim 1 or 2.

**19.** The antibody-drug conjugate according to claim 18, **characterized in that** the conjugate moiety comprises a detectable label, a chemical drug, or a cytotoxin.

EP 4 763 863 A1

Figure 1

Figure 2

18

Figure 3

## INTERNATIONAL SEARCH REPORT

| International application No. |
|---|
| **PCT/CN2024/103547** |

**A. CLASSIFICATION OF SUBJECT MATTER**

C07K16/28(2006.01)i; C07K16/30(2006.01)i; C12N15/13(2006.01)i; A61K39/395(2006.01)i; A61P35/00(2006.01)i; A61P35/02(2006.01)i; G01N33/574(2006.01)i

According to International Patent Classification (IPC) or to both national classification and IPC

**B. FIELDS SEARCHED**

Minimum documentation searched (classification system followed by classification symbols)

IPC: C07K C12N A61K A61P G01N

Documentation searched other than minimum documentation to the extent that such documents are included in the fields searched

Electronic data base consulted during the international search (name of data base and, where practicable, search terms used)

CNABS, DWPI, SIPOABS, CNTXT, EPTXT, USTXT, WOTXT, PUBMED, CNKI, NCBI, EBI, STN, 中国专利生物序列检索系统, China Patent Biological Sequence Search System, 百度学术, Baidu Scholar: 申请人, Applicants, 发明人, Inventors, BCMA, 单域抗体, 纳米抗体, VHH, SEQ ID NOs: 1-4

**C. DOCUMENTS CONSIDERED TO BE RELEVANT**

| Category* | Citation of document, with indication, where appropriate, of the relevant passages | Relevant to claim No. |
|---|---|---|
| PX | CN 117384287 A (ZHEJIANG CARBIOGENE THERAPEUTICS CO., LTD.) 12 January 2024 (2024-01-12) <br> claims 1-10, and description, paragraphs 72-73 | 1-19 |
| A | WO 2022089353 A1 (NANJING BIOHENG THERAPEUTICS CO., LTD. et al.) 05 May 2022 (2022-05-05) <br> abstract, and claims 1-26 | 1-19 |
| A | WO 2022199590 A1 (ZHEJIANG NANOMAB TECHNOLOGY CENTER CO., LTD.) 29 September 2022 (2022-09-29) <br> abstract, and claims 1-12 | 1-19 |
| A | WO 2022033057 A1 (SHENZHEN WINGOR BIO-TECHNOLOGY CO., LTD.) 17 February 2022 (2022-02-17) <br> abstract, and claims 1-10 | 1-19 |
| A | WO 2023098846 A1 (JIANGSU SIMCERE PHARMACEUTICAL CO., LTD.) 08 June 2023 (2023-06-08) <br> abstract, and claims 1-22 | 1-19 |

☑ Further documents are listed in the continuation of Box C. ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **13 September 2024** | **26 September 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/103547**

**C.     DOCUMENTS CONSIDERED TO BE RELEVANT**

| Category* | Citation of document, with indication, where appropriate, of the relevant passages | Relevant to claim No. |
|---|---|---|
| A | WO 2021043169 A1 (ABLINK BIOTECH CO., LTD. et al.) 11 March 2021 (2021-03-11) abstract, and claims 1-22 | 1-19 |
| A | CN 111201243 A (MOGAM INSTITUTE FOR BIOMEDICAL RESEARCH et al.) 26 May 2020 (2020-05-26) abstract, and claims 1-13 | 1-19 |

**INTERNATIONAL SEARCH REPORT**

International application No.

**PCT/CN2024/103547**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
|---|
| **PCT/CN2024/103547** |

**Box No. II    Observations where certain claims were found unsearchable (Continuation of item 2 of first sheet)**

This international search report has not been established in respect of certain claims under Article 17(2)(a) for the following reasons:

1. ☑   Claims Nos.: **5-7 (in part), 13, 14-16 (in part)**
because they relate to subject matter not required to be searched by this Authority, namely:

Claims 5-7 (in part), 13 and 14-16 (in part) relate to the use or method of a single-domain antibody or a biological material in preventing or treating tumors, which falls within subject matter for which no search is required by the International Searching Authority as defined in PCT Rule 39.1. The international search is carried out on the basis of the use of a single-domain antibody or a biological material in the preparation of a drug for preventing or treating BCMA target-related diseases.

2. ☐   Claims Nos.:
because they relate to parts of the international application that do not comply with the prescribed requirements to such an extent that no meaningful international search can be carried out, specifically:

3. ☐   Claims Nos.:
because they are dependent claims and are not drafted in accordance with the second and third sentences of Rule 6.4(a).

Form PCT/ISA/210 (continuation of first sheet) (July 2022)

23

## INTERNATIONAL SEARCH REPORT
### Information on patent family members

| International application No. |
| --- |
| **PCT/CN2024/103547** |

| Patent document cited in search report | | | Publication date (day/month/year) | Patent family member(s) | | | Publication date (day/month/year) |
| --- | --- | --- | --- | --- | --- | --- | --- |
| CN | 117384287 | A | 12 January 2024 | None | | | |
| WO | 2022089353 | A1 | 05 May 2022 | US | 2024018251 | A1 | 18 January 2024 |
| WO | 2022199590 | A1 | 29 September 2022 | EP | 4317186 | A1 | 07 February 2024 |
| | | | | US | 2024174760 | A1 | 30 May 2024 |
| WO | 2022033057 | A1 | 17 February 2022 | CN | 111909271 | A | 10 November 2020 |
| | | | | CN | 111909271 | B | 23 March 2021 |
| WO | 2023098846 | A1 | 08 June 2023 | None | | | |
| WO | 2021043169 | A1 | 11 March 2021 | CN | 114667294 | A | 24 June 2022 |
| CN | 111201243 | A | 26 May 2020 | KR | 20200032218 | A | 25 March 2020 |
| | | | | KR | 102403046 | B1 | 30 May 2022 |
| | | | | AU | 2018341541 | A1 | 07 May 2020 |
| | | | | AU | 2018341541 | B2 | 23 December 2021 |
| | | | | EP | 3689908 | A2 | 05 August 2020 |
| | | | | EP | 3689908 | A4 | 29 September 2021 |
| | | | | WO | 2019066435 | A2 | 04 April 2019 |
| | | | | WO | 2019066435 | A3 | 06 June 2019 |
| | | | | WO | 2019066435 | A9 | 18 July 2019 |
| | | | | JP | 2020534841 | A | 03 December 2020 |
| | | | | JP | 7137619 | B2 | 14 September 2022 |
| | | | | BR | 112020006248 | A2 | 20 October 2020 |
| | | | | BR | 112020006248 | A8 | 21 March 2023 |
| | | | | CA | 3076972 | A1 | 04 April 2019 |
| | | | | CA | 3076972 | C | 26 September 2023 |
| | | | | US | 2020299395 | A1 | 24 September 2020 |
| | | | | US | 11447559 | B2 | 20 September 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311320141

**Non-patent literature cited in the description**

- Remington: The Science and Practice of Pharmacy. Pharmaceutical Press, 2012